(19) Europäisches Patentamt
European Patent Office
Office européen des brevets

(11) **EP 4 311 809 A1**

(12) **EUROPÄISCHE PATENTANMELDUNG**

(43) Veröffentlichungstag:
**31.01.2024   Patentblatt 2024/05**

(21) Anmeldenummer: **22186943.1**

(22) Anmeldetag: **26.07.2022**

(51) Internationale Patentklassifikation (IPC):
*C02F 1/34* *(2023.01)*          *A61L 2/02* *(2006.01)*
*B01D 21/28* *(2006.01)*          *B06B 1/06* *(2006.01)*
*C02F 1/52* *(2023.01)*          *C12N 13/00* *(2006.01)*
*A61L 2/025* *(2006.01)*          *C02F 1/36* *(2023.01)*

(52) Gemeinsame Patentklassifikation (CPC):
**C02F 1/36; B01D 21/283; C02F 1/5209;**
**C12N 13/00;** B01D 2221/02; B06B 1/0607;
C02F 2201/004; C02F 2301/063; C02F 2303/04;
C02F 2307/14

(84) Benannte Vertragsstaaten:
**AL AT BE BG CH CY CZ DE DK EE ES FI FR GB**
**GR HR HU IE IS IT LI LT LU LV MC MK MT NL NO**
**PL PT RO RS SE SI SK SM TR**
Benannte Erstreckungsstaaten:
**BA ME**
Benannte Validierungsstaaten:
**KH MA MD TN**

(71) Anmelder: **Georg Fischer JRG AG**
**4450 Sissach (CH)**

(72) Erfinder:
• **Nguyen, Minh Hop**
**4614 Haegendorf (CH)**
• **Lehmann, Steffen**
**79713 Bad Saeckingen (DE)**

• **Pavlic, Alen**
**8912 Obfelden (CH)**
• **Camelin, Enrico**
**4460 Gelterkinden (CH)**
• **Dual, Juerg**
**8352 Elsau (CH)**
• **Buerli, Stephan**
**4451 Wintersingen (CH)**
• **Rosenthaler, Lucas Raphael**
**4106 Therwil (CH)**

(74) Vertreter: **Fenner, Seraina**
**Georg Fischer AG**
**Amsler-Laffon-Strasse 9**
**8201 Schaffhausen (CH)**

(54) **VORRICHTUNG ZUR SEPARATION VON LEGIONELLEN**

(57)    Vorrichtung (1) zur Konzentration und Separation von Legionellen und/oder Amöben durch Akustophorese in Leitungswasser beinhaltend, eine Strömungskammer (2) mit einer Einlassöffnung (5) durch die das Leitungswasser hineinströmt und einer Auslassöffnung (6) durch die das Leitungswasser hinausströmt, wobei sich die Öffnungen gegenüberliegen und einer Öffnung (7) zur Ableitung konzentrierter Legionellen und/oder Amöben, wobei die Strömungskammer Abmasse aufweist, vorzugsweise eine Länge (L), eine Höhe (H) und eine Breite (B), und mindestens zwei ausserhalb der Strömungskammer, an jeweils zwei Seiten der Strömungskammer, angeordnete Wandler (3) zur Anwendung akustischer Energie auf die Strömungskammer zur Erzeugung von stehenden Wellen, wobei mindestens eines der Abmasse der Strömungskammer unter Berücksichtigung des akustischen Kontrast Faktors $\Phi$ der Legionellen und/oder Amöben derart ausgelegt ist, um eine oder mehrere Frequenzen und Druckamplituden der stehenden Wellen zu erzeugen, dass sich die Legionellen und/oder Amöben in den Druckknoten der stehenden Welle konzentrieren bzw. ansammeln und durch die Rohrleitung der Ableitung z. B. entfernt werden können.

Fig. 1

EP 4 311 809 A1

**Beschreibung**

Die Erfindung betrifft eine Vorrichtung zur Konzentration und Separation von Legionellen und/oder Amöben durch Akustophorese in Leitungswasser, vorzugsweise Trinkwasser beinhaltend, eine Strömungskammer mit einer Einlassöffnung durch die das Leitungswasser hineinströmt und einer Auslassöffnung durch die das Leitungswasser hinausströmt, wobei sich die Öffnungen gegenüberliegen und einer Öffnung zur Ableitung konzentrierter Legionellen und/oder Amöben, wobei die Strömungskammer Abmasse aufweist, vorzugsweise eine Länge, eine Höhe und eine Breite, und mindestens zwei ausserhalb der Strömungskammer, an jeweils zwei Seiten der Strömungskammer, angeordnete Wandler zur Anwendung akustischer Energie auf die Strömungskammer zur Erzeugung von stehenden Wellen.

Die Akustophorese ist eine Methode, um Teilchen in einem Medium unter Verwendung des Strahlungsdrucks von intensiven Schallwellen zu konzentrieren und/oder zu separieren. Die Teilchen können z.B. Schmutzpartikel, Bakterien, Legionellen oder Amöben sein. Für die Generierung des Strahlungsdrucks können stehende Schallwellen induziert werden, welche Kräfte auf Partikel ausüben. Diese Methode ist ebenfalls in der Akustofluidik bekannt und wird seit mehreren Jahrzehnten angewandt. Das Druckprofil einer stehenden Welle variiert zwischen Bereichen mit hohem Druck bzw. Druckdifferenz und Bereichen mit niedrigem akustischem Druck, wobei der niedrige Bereich in den Druckknoten zu finden ist. Solche stehenden Wellen dienen also dazu um Partikel in einem Medium zu konzentrieren und/oder zu separieren.

Die WO2013/138797 offenbart ein Verfahren zum Trennen von Verunreinigungen in einem Host-Fluid, wobei die Verunreinigungen Partikel jeglicher Grösse sind, die mit dem offenbarten Verfahren separiert werden sollen. Entsprechend der Partikelgrössen werden die Ultraschallwellen angepasst.

Es ist Aufgabe der Erfindung eine Vorrichtung vorzuschlagen, welche auf die Konzentration und Separation von Legionellen und/oder Amöben in Leitungswasser ausgelegt ist.

Diese Aufgabe wird erfindungsgemäss dadurch gelöst, dass mindestens eines der Abmasse der Strömungskammer unter Berücksichtigung des akustischen Kontrast Faktors Φ der Legionellen und/oder Amöben derart ausgelegt ist, um eine oder mehrere Frequenzen und Druckamplituden der stehenden Wellen zu erzeugen, dass sich die Legionellen und/oder Amöben in den Druckknoten der stehenden Welle konzentrieren bzw. ansammeln.

Der akustische Kontrast Faktor Φ ist wie folgt definiert:

$$\Phi = \frac{1}{3}\left[\frac{5\rho_p - 2\rho_0}{2\rho_p + \rho_0} - \frac{\kappa_p}{\kappa_0}\right]$$

und somit Abhängig der Dichte $\rho_0$ und der Kompressibilität $\kappa_0$ des Leitungswassers, wie auch Abhängig der Dichte $\rho_p$ und der Kompressibilität $\kappa_p$ des Partikels, sprich jene der Legionellen und den Amöben.

Die erfindungsgemässe Vorrichtung zur Konzentration und Separation von Legionellen und/oder Amöben durch Akustophorese in Leitungswasser beinhaltet mindestens zwei ausserhalb der Strömungskammer an jeweils zwei Seiten der Strömungskammer angeordnete Wandler zur Anwendung akustischer Energie auf die Strömungskammer. Die Wandler sind vorzugsweise jeweils in der gleichen Ebene wie auch versetzt zueinander senkrecht zur Strömungsrichtung an jeweils zwei Seiten der Strömungskammer positioniert. Dadurch werden mindestens zwei stehende Schallwellen in der Strömungskammer, welche von Leitungswasser durchströmt wird, erzielt.

Als Wandler kann ein Ultraschallwandler vorzugsweise mit einem Piezoelement oder vorzugweise mit einem geschichteten Piezoelement dienen. Als Wandler können aber auch Antriebe/Aktuatoren eingesetzt werden, wie Schwingspulen oder Exzentertriebe.

Die erfindungsgemässe Vorrichtung weist eine Öffnung zur Ableitung der Legionellen und/oder Amöben auf. Vorteilhaft ist es, wenn daran eine Leitung angeschlossen ist, die es ermöglicht die konzentrierten Legionellen und/oder Amöben gleich abzuleiten.

Die Strömungsgeschwindigkeit des Leitungswassers mit den konzentrierten Legionellen und/oder Amöben in der Ableitung entspricht vorzugsweise mindestens der Strömungsgeschwindigkeit in der Strömungskammer. Vorzugsweise ist ein Unterdruck in der Ableitung im Vergleich zum Druck in der Strömungskammer vorhanden.

Die Strömungskammer der erfindungsgemässen Vorrichtung weist Abmasse auf, vorzugsweise eine Länge, eine Breite und eine Höhe, wobei mindestens eines der Abmasse der Strömungskammer unter Berücksichtigung des akustischen Kontrast Faktors Φ der Legionellen und/oder Amöben derart ausgelegt ist, um eine oder mehrere Frequenzen und Druckamplituden der stehenden Wellen zu erzeugen, dass sich die Legionellen und/oder Amöben in den Druckknoten der stehenden Welle konzentrieren bzw. ansammeln.

Die Abmasse bzw. eines dieser Abmasse, wie die Höhe, Breite und Länge der Strömungskammer sind derart optimiert, dass ein Minimum an Energie aufgewendet werden muss, um die Legionellen und/oder Amöben im Leitungswasser zu konzentrieren und zu separieren.

Als vorteilhaft hat sich gezeigt, wenn die Höhe und die Breite derart ausgelegt sind, dass die Druckknoten, welche entsprechend den durch die Wandler erzeugten stehenden Wellen entstehen, in einer Linie mit der Öffnung zur Ableitung der Legionellen und/oder Amöben liegen und sich vorzugsweise zumindest über einen Teil der Länge der Strömungskammer erstrecken. Dadurch können Legionellen und/oder Amöben einfach separiert werden, da sie sich in den Druckknoten ansammeln und in einer Linie bis zur und dann durch die Öffnung abgeleitet werden.

Als bevorzugte Ausführung hat sich gezeigt, wenn die Strömungskammer eine rechteckige Querschnittsfläche aufweist.

Als vorteilhaft hat sich gezeigt, dass mindestens an einer Seite der Höhe und mindestens an einer Seite der Breite jeweils mindestens ein Wandler angeordnet ist, um die Strömungskammer von zwei Seiten anzuregen. Dadurch überlagern sich die Druckfelder der erzeugten stehenden Wellen. Die zwei Wellen können so jeweils in unterschiedlichen Separationsabständen entlang der Länge der Strömungskammer Druckknoten haben. Vorzugsweise sind die Wandler jeweils in der Mitte einer Seite des Querschnitts angeordnet.

Vorzugsweise sind die Abmasse der Höhe und die Breite der Strömungskammer unterschiedlich lang ausgebildet. Dadurch wird erreicht, dass sich die Druckfelder, aufgrund der an den Seiten angeordneten Wandler, wobei ein Wandler an einer Seite der Breite und die andere Quelle an einer Seite der Höhe angeordnet ist, sich überlagern und die Legionellen und/oder Amöben auch von den Ecken der Strömungskammer in die Mitte bzw. in die Druckknoten gelenkt werden.

Vorzugsweise generieren die Wandler einen zusätzlichen akustischen Druck bzw. Druckdifferenz in der Strömungskammer von mindestens 5 MPa, vorzugsweise 10 MPa und mehr. Vorzugsweise wird die Umgebung einer Resonanzfrequenz der Strömungskammer gewählt, welche vorzugsweise aus einem niedrigdämpfenden Material gebaut wird. Dadurch wird ein minimaler Energieaufwand benötigt. Vorzugsweise wird dazu eine Frequenz zwischen 15 kHz und 150kHz von den Wandlern aufgebracht.

Es ist vorteilhaft, wenn die Strömungskammer eine Höhe und eine Breite aufweist, mittels denen die durch die Wandler erzeugten Frequenzen einer stehenden Welle generiert werden, die Druckknoten im Zentrum des Querschnitts der Strömungskammer aufweisen. Es ist vorteilhaft gegenüber alternativen Separationsmethoden wie Ultrafiltration, chemische Behandlung etc., die Legionellen und Amoeben so berührungsfrei und nicht invasiv zu konzentrieren und zu separieren, um so invasive Eingriffe in einem Leitungswassersystem zu verhindern.

Als bevorzugte Ausführungsform hat sich gezeigt, wenn die stehenden Wellen in der Strömungskammer im Bereich von 15 kHz bis 150 kHz liegen, vorzugsweise 20 kHz bis 60 kHz. Vorzugsweise wird die Umgebung einer Resonanzfrequenz der Strömungskammer gewählt, welche vorzugsweise aus einem niedrigdämpfenden Material gebaut wird.

Vorteilhaft ist es, wenn die Länge der Strömungskammer umgekehrt proportional zum akustischen Kontrastfaktor $\Phi$ der Legionellen und/oder Amöben ist. Das heisst je kleiner der akustische Kontrastfaktor $\Phi$ der Legionellen und/oder Amöben ist desto länger muss die Strömungskammer sein. Zudem skaliert sich der nötige akustische Druck in der Strömungskammer umgekehrt proportional zur Wurzel des akustischen Kontrastfaktors $\Phi$ der Legionellen und/oder Amöben. Das heisst je kleiner der akustische Kontrastfaktor $\Phi$ der Legionellen und/oder Amöben ist, desto grösser muss der akustische Druck in der Strömungskammer sein um den gewünschten Effekt der Ansammlung der Legionellen und/oder Amöben an den Druckknoten zu erzielen.

Vorzugsweise beträgt die Länge, abhängig vom Volumenstrom, der Druckamplitude und dem akustischen Kontrastfaktor $\Phi$ der Legionellen und/oder Amöben, der Strömungskammer 15 mm bis 150 cm. Es hat sich gezeigt, dass über diese Distanzen, abhängig von Frequenzen, Druckamplituden und akustischem Kontrastfaktor $\Phi$ der Legionellen und/oder Amöben die Legionellen und/oder Amöben genügend Zeit haben, um sich in den Druckknoten anzusammeln.

Als vorteilhaft hat sich gezeigt, wenn die Höhe der Strömungskammer mindestens 16 mm und die Breite mindestens 16 mm beträgt. Neben der Abstimmung auf den akustischen Kontrastfaktor wird auch erreicht, dass die Strömungskammer an einer Rohrleitung mit einem Durchmesser von 16 mm problemlos anschliessbar ist.

Vorzugsweise weist die Strömungskammer eine variable Wandstärke mit Vertiefungen und Verdickungen von mindestens 1mm bis maximal 10 mm entlang der Strömungskammer auf. Dadurch ist die Wand nicht plan ausgebildet, sondern weist eine strukturierte Oberfläche auf.

Die Radien in den Wandübergängen auf der Innenseite der Struktur der Strömungskammer betragen mindestens 1/200 der Querschnittsdimensionen (B und H).

Als vorteilhaft hat sich gezeigt, wenn das Material der Strömungskammer aus einem mit Leitungswasser verträglichen Material hergestellt ist, welches zudem akustische Wellen reflektiert und wenig absorbiert, wie vorzugsweise Kupferlegierungen wie Rotguss, Messing oder nichtrostende Stähle.

Diese Wandstärke und auch die erwähnten Materialen ermöglichen ein gutes Übertragen der akustischen Energie vom Wandler auf das Leitungswasser und bringt zudem der Strömungskammer auch ausreichend Stabilität, um die Anforderungen von Wasserleitungen zu erfüllen.

Es ist vorteilhaft, wenn die Wandler senkrecht zur Längsachse der Strömungskammer ausgerichtet und auf der gleichen Ebene wie auch auf unterschiedlichen Ebenen entlang der Längsachse der Strömungskammer angeordnet sind. Das heisst, dass die Wandler entlang der Längsachse auf unterschiedlichen Positionen angeordnet sein können wie auch auf derselben Höhe entlang der Längsachse.

Vorzugsweise werden die Wandler mit einer Masse bzw. dem Schwingkolben verbunden, der wiederum über ein Federelement wie beispielsweise einem Gummipuffer mit der Strömungskammer verbunden ist, um so die akustische Energie vom Wandler in das Leitungswasser zu führen. Dabei wird der Wandler in seiner Wirkrichtung auf dem Schwingkolben angebracht, dieser hat Kontakt mit dem Leitungswasser in der Strömungskammer und ist über das Federelement mit der mechanischen Struktur der Strömungskammer verbunden. Die Breite der Kontaktfläche des Schwingkolbens mit dem Leitungswasser ist vorzugsweise mindestens 40% so breit wie die innere Querschnittsbreite der Strömungskammer senkrecht zur Wirkrichtung des jeweiligen Wandlers. Als bevorzugte Ausführungsform hat sich auch gezeigt, wenn die Länge des Kolbens abhängig von der Masse und der Länge der Strömungskammer, mindestens 10 mm lang ist. Der Schwingkolben besteht aus denselben Materialien wie jene die in der Strömungskammer verwendet werden. Die Masse des Schwingkolben beträgt entsprechend den wie in der Strömungskammer verwendeten Materialen, wie beschrieben entsprechend der Geometrie der Strömungskammer. Das Feder-Massen-System bestehend aus Wandler, Schwingkolben und Federelement und ohne Wasserkontakt hat vorzugweise eine Resonanzfrequenz von 50 Hz zu 150 KHz. Als vorteilhaft hat sich gezeigt, dass das Federelement aus Leitungswasserverträglichem Material wie z.B. EPDM besteht und zwischen Schwingkolben und Leitungswasser totraumfrei abdichtet. Vorzugsweise weist es eine niedrige Dämpfung zwischen 0.5% und 30% auf und einen E-Modul von zwischen 30 MPa und 30 GPa hat.

Die Strömungskammer der erfindungsgemässen Vorrichtung weist eine Einlass- und eine Auslassöffnung auf durch die das Leitungswasser herein und wieder hinausströmt. Die Öffnungen liegen sich gegenüber, vorzugsweise so, dass die Strömungskammer in eine Rohrleitung integriert werden kann. Die erfindungsgemässe Vorrichtung wird über die Einlass- und die Auslassöffnungen mit der Rohrleitung für das Trinkwasser verbunden.

Ein Ausführungsbeispiel der Erfindung wird anhand der Figuren beschrieben, wobei sich die Erfindung nicht nur auf das Ausführungsbeispiel beschränkt. Es zeigen:

Fig. 1    eine schematische Darstellung einer erfindungsgemässen Vorrichtung,

Fig. 2    den Querschnitt einer Strömungskammer und

Fig. 3    ein Feder-Masse-System zur Anbindung des Wandlers an der Strömungskammer

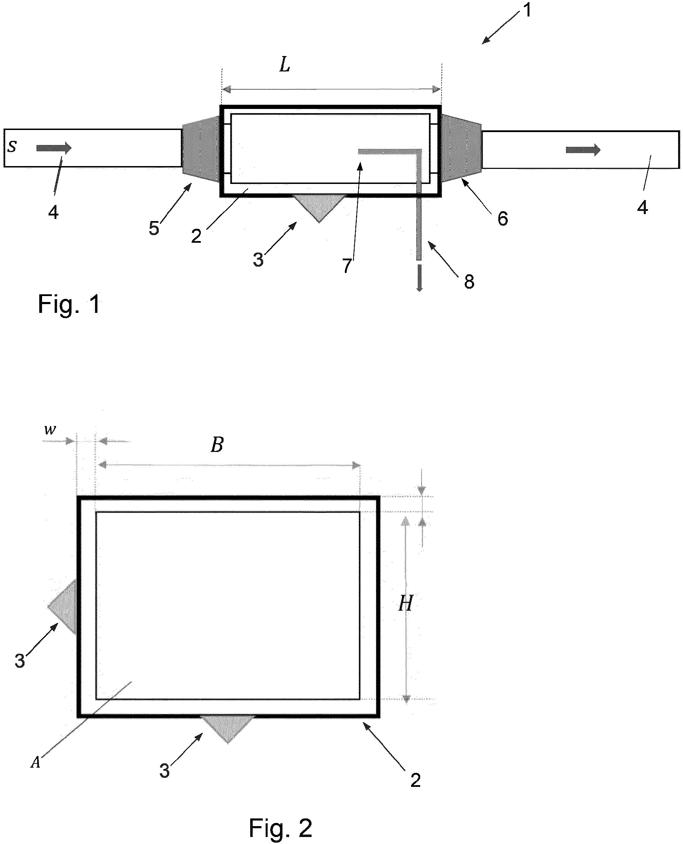

Die in Fig. 1 dargestellte Zeichnung zeigt eine schematische Darstellung einer erfindungsgemässen Vorrichtung 1. Die Vorrichtung zur Konzentration und Separation von Legionellen und/oder Amöben durch Akustophorese ist in einer Wasserleitung 4 angeordnet. Die Vorrichtung 1 weist eine Strömungskammer 2 auf die jeweils an den beiden Stirnseiten über Ein- und Auslassöffnungen 5, 6 mit der Rohrleitung 4 verbunden ist. Das Leitungswasser durchströmt entsprechend die Strömungskammer 2. An mindestens zwei Seiten der Strömungskammer 2 sind die Wandler 3 zur Erzeugung der akustischen Energie angeordnet. Wobei jeweils ein Wandler 3 an einer Seite der Breite B und ein Wandler 3 an einer Seite der Höhe H angeordnet ist. Durch die über die Wandler 3 erzeugten Schallwellen, werden aufgrund des rechteckigen Querschnitts, wie in Fig. 2 ersichtlich ist, Druckfeldüberlagerungen erzielt und dadurch, dass die Höhe H und die Breite B unterschiedliche Längen aufweisen, werden auch die Legionellen und/oder Amöben aus den Ecken in die Mitte zu den Druckknoten getragen bzw. geleitet. Die in den Druckknoten angesammelten Legionellen und/oder Amöben werden dann über eine Öffnung 7 zur Ableitung der Legionellen und/oder Amöben geleitet, welche ebenso vorzugsweise auf einer Linie mit den Druckknoten liegt. An die Öffnung 7 schliesst vorzugsweise eine Rohrleitung 8 zur Ableitung der Legionellen und/oder Amöben an. Als vorteilhaft hat sich gezeigt, wenn die Höhe H und die Breite B derart ausgelegt sind, dass der akustische Druck bzw. Druckdifferenz in der Strömungskammer 2 mindestens 5 MPa ist, vorzugsweise 10 MPa und höher. Wobei der Wandler 3.1 und 3.2 vorzugsweise in der Umgebung einer Resonanzfrequenz der Strömungskammer betrieben werden, welche vorzugsweise aus einem niedrigdämpfenden Material gebaut wird, und so ein möglichst geringer Energiebedarf aufgewendet wird. Vorzugsweise haben die Schallwellen im Leitungswasser eine Frequenz von 15kHz bis 150kHz ausbreiten. Vorzugsweise bilden sich dadurch im Zentrum des Querschnitts A die Druckknoten, welches sich zumindest über einen Teil der Länge L der Strömungskammer 2 erstreckt. Es ist vorteilhaft wenn im Zentrum ein mindestens akustischer Druck bzw. Druckdifferenz von 0 MPa vorliegt, wodurch sich die Legionellen und/oder Amöben dort ansammeln und in die Öffnung 7 geleitet werden.

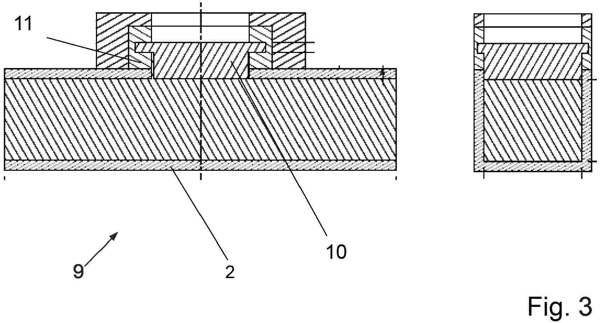

In Fig. 3 ist das Feder-Massen-System 9 abgebildet welches die Verbindung zwischen der Strömungskammer 2 und dem Wandler 3 darstellt. Der Schwingkolben weist vorzugsweise eine Breite von mindestens 40% der Breite der Strömungskammer auf, wobei die dargestellte Ausführungsform die komplette Breite abdeckt. Der Kolben 10 kontaktier das sich in der Strömungskammer befindende Leitungswasser. Der Schwingkolben 10 ist in einem Federelement 11 gelagert, das mit der Strömungskammer 2 verbunden ist.

Bezugszeichenliste

1    Vorrichtung
2    Strömungskammer

3    Wandler
4    Wasserleitung
5    Einlassöffnung
6    Auslassöffnung
7    Öffnung zur Ableitung
8    Rohrleitung der Ableitung
9    Feder-Massen-System
10   Schwingkolben
11   Federelement

L    Länge Strömungskammer
H    Höhe Strömungskammer
B    Breite Strömungskammer
W    Wandstärke Strömungskammer
s    Strömung des Leitungswassers
A    Querschnittsfläche der Strömungskammer

**Patentansprüche**

1. Vorrichtung (1) zur Konzentration und Separation von Legionellen und/oder Amöben durch Akustophorese in Leitungswasser (4) beinhaltend, eine Strömungskammer (2) mit einer Einlassöffnung (5) durch die das Leitungswasser hineinströmt und einer Auslassöffnung (6) durch die das Leitungswasser hinausströmt, wobei sich die Öffnungen (5, 6) gegenüberliegen und einer Öffnung (7) zur Ableitung konzentrierter Legionellen und/oder Amöben, wobei die Strömungskammer (2) Abmasse aufweist, vorzugsweise eine Länge (L), eine Höhe (H) und eine Breite (B), und mindestens zwei ausserhalb der Strömungskammer, (2) an jeweils zwei Seiten der Strömungskammer (2), angeordnete Wandler (3) zur Anwendung akustischer Energie auf die Strömungskammer (2) zur Erzeugung von stehenden Wellen, **dadurch gekennzeichnet dass**, mindestens eines der Abmasse der Strömungskammer unter Berücksichtigung des akustischen Kontrast Faktors Φ der Legionellen und/oder Amöben derart ausgelegt ist, um eine oder mehrere Frequenzen und Druckamplituden der stehenden Wellen zu erzeugen, dass sich die Legionellen und/oder Amöben in den Druckknoten der stehenden Welle konzentrieren bzw. ansammeln.

2. Vorrichtung (1) gemäss Anspruch 1, **dadurch gekennzeichnet, dass** mindestens eines der Abmasse der Strömungskammer unter Berücksichtigung des akustischen Kontrast Faktors Φ der Legionellen und/oder Amöben derart ausgelegt ist, um eine oder mehrere Frequenzen und Druckamplituden der stehenden Wellen zu erzeugen, dass die Druckknoten, die durch die Wandler erzeugten stehenden Wellen, in einer Linie mit der Öffnung zur Ableitung der Legionellen und/oder Amöben liegen.

3. Vorrichtung gemäss Anspruch 1 oder 2 **dadurch gekennzeichnet, dass** die Strömungskammer eine rechteckige Querschnittsfläche aufweist.

4. Vorrichtung gemäss Anspruch 1 bis 3 **dadurch gekennzeichnet, dass** die Höhe H und die Breite B der Strömungskammer unterschiedlich lang sind.

5. Vorrichtung gemäss Anspruch 1 bis 4 **dadurch gekennzeichnet, dass** die Höhe (H) und die Breite (B) der Strömungskammer (2) derart ausgelegt sind, dass mittels den erzeugten stehenden Wellen, ein akustischer Druck bzw. Druckdifferenz von mindestens 5 MPa, vorzugsweise 10 MPa und höher in der Strömungskammer (2) generieren, vorzugsweise bei einer erzeugten Frequenz von 15 kHz bis 150 kHz.

6. Vorrichtung (1) gemäss Anspruch 1 bis 5 **dadurch gekennzeichnet, dass** mindestens eines der Abmasse der Strömungskammer unter Berücksichtigung des akustischen Kontrast Faktors Φ der Legionellen und/oder Amöben derart ausgelegt ist, um eine oder mehrere Frequenzen und Druckamplituden der stehenden Wellen zu erzeugen, die einen minimalen akustischen Druck bzw. Druckdifferenz im Zentrum des Querschnitts (A) der Strömungskammer (2) von 0 MPa aufweist.

7. Vorrichtung (1) gemäss Anspruch 1 bis 6 **dadurch gekennzeichnet, dass** die stehenden Wellen in der Strömungskammer (2) im Bereich von 15 kHz bis 150 kHz liegen, vorzugsweise in der Umgebung einer Resonanzfrequenz der Strömungskammer, welche vorzugsweise aus einem niedrigdämpfenden Material gebaut wird, und so einen möglichst geringen Energiebedarf erfordert.

8. Vorrichtung (1) gemäss Anspruch 1 bis 7 **dadurch gekennzeichnet, dass** der maximale akustische Druck bzw. Druckdifferenz der Strömungskammer (2) umgekehrt proportional zur Wurzel des akustischen Kontrastfaktors Φ der Legionellen und/oder Amöben ist.

9. Vorrichtung (1) gemäss Anspruch 1 bis 8 **dadurch gekennzeichnet, dass** die Länge (L) der Strömungskammer 15mm bis 150cm beträgt.

10. Vorrichtung (1) gemäss Anspruch 1 bis 9 **dadurch gekennzeichnet, dass** die Höhe (H) der Strömungskammer mindestens 16 mm und die Breite (B) mindestens 16 mm beträgt.

11. Vorrichtung (1) gemäss Anspruch 1 bis 10 **dadurch gekennzeichnet, dass** die Strömungskammer (2) eine variable Wandstärke (w) mit Vertiefungen und Materialverdickungen von mindestens 1 mm und maximal 10 mm aufweist.

**12.** Vorrichtung (1) gemäss Anspruch 1 bis 11 **dadurch gekennzeichnet, dass** die Strömungskammer (2) aus einem mit Leitungswasser verträglichen Material hergestellt ist, welches zudem akustische Wellen reflektiert und wenig absorbiert, wie vorzugsweise Kupferlegierungen speziell bevorzugt Rotguss, Messing, oder nichtrostende Stähle.

**13.** Vorrichtung (1) gemäss Anspruch 1 bis 12 **dadurch gekennzeichnet, dass** die Wandler senkrecht zur Längsachse der Strömungskammer ausgerichtet und auf der gleichen Ebene wie auch auf unterschiedlichen Ebenen entlang der Längsachse der Strömungskammer (2) angeordnet sind.

**14.** Vorrichtung (1) gemäss Anspruch 1 bis 13 **dadurch gekennzeichnet, dass** die Wandler (3) aus einem Piezoelement oder einem geschichteten Piezoelement gebildet sind.

**15.** Vorrichtung (1) gemäss Anspruch 1 bis 14 **dadurch gekennzeichnet, dass** die Wandler mit einer Masse bzw. einem Schwingkolben (10) verbunden ist, wobei der Schwingkolben (10) über ein Federelement (11) mit der Strömungskammer (2) verbunden sind.

Fig. 1

Fig. 2

11

9

2

10

Fig. 3

Europäisches
Patentamt

European
Patent Office

Office européen
des brevets

# EUROPÄISCHER RECHERCHENBERICHT

**Nummer der Anmeldung**

EP 22 18 6943

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2011/126371 A2 (STICHTING WETSUS CT EXCELLENCE SUSTAINABLE WATER TECHNOLOGY [NL] ET AL) 13. Oktober 2011 (2011-10-13) * Abbildungen 1-3 * * Seite 1, Zeilen 4-28 * * Seite 2, Zeilen 12-17 * * Seite 4, Zeile 16 - Seite 6, Zeile 4 * * Seite 10, Zeile 18 - Seite 12, Zeile 28 * ----- | 1-15 | INV. C02F1/34 A61L2/02 B01D21/28 B06B1/06 C02F1/52 C12N13/00 A61L2/025 C02F1/36 |
| X | CN 204 563 692 U (UNIV SHAANXI NORMAL) 19. August 2015 (2015-08-19) * Abbildungen 1-2 * * Zusammenfassung * * Absätze [0014] - [0026] * ----- | 1-15 | |
| X | US 2017/291122 A1 (LIPKENS BART [US] ET AL) 12. Oktober 2017 (2017-10-12) * Abbildungen 1b, 10a, * * Absätze [0008] - [0014], [0087] - [0102], [0117] - [0122], [0132] - [0139] * ----- | 1-15 | RECHERCHIERTE SACHGEBIETE (IPC) C02F C12R B06B B01D A61L C12N |
| X | US 2017/128857 A1 (LIPKENS BART [US] ET AL) 11. Mai 2017 (2017-05-11) * Abbildungen 5-6 * * Absätze [0002], [0006] - [0020], [0059] - [0075] * ----- | 1-15 | |
| X | WO 2011/130321 A2 (FLODESIGN SONICS INC [US]; DIONNE JASON [US] ET AL.) 20. Oktober 2011 (2011-10-20) * Abbildungen 3-4 * * Absätze [0003] - [0016], [0019] - [0020], [0033] - [0085] * ----- | 1-15 | |

-/--

Der vorliegende Recherchenbericht wurde für alle Patentansprüche erstellt

| Recherchenort | Abschlußdatum der Recherche | Prüfer |
|---|---|---|
| Den Haag | 21. Dezember 2022 | Rozanska, Agnieszka |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

Seite 1 von 2

**EUROPÄISCHER RECHERCHENBERICHT**

Nummer der Anmeldung

EP 22 18 6943

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2011/123392 A1 (DIONNE JASON [US] ET AL) 26. Mai 2011 (2011-05-26) <br> * Abbildungen 1,9 * <br> * Absätze [0002] – [0064] * <br> ----- | 1-15 | |
| A | FRANCISCO J. TRUJILLO ET AL: "Multiphysics modelling of the separation of suspended particles via frequency ramping of ultrasonic standing waves", ULRASONICS SONOCHEMISTRY, Bd. 20, Nr. 2, 13. September 2012 (2012-09-13), Seiten 655-666, XP055325026, GB ISSN: 1350-4177, DOI: 10.1016/j.ultsonch.2012.08.014 * das ganze Dokument * <br> ----- | 1-15 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| Den Haag | 21. Dezember 2022 | Rozanska, Agnieszka |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

Seite 2 von 2

**ANHANG ZUM EUROPÄISCHEN RECHERCHENBERICHT**
**ÜBER DIE EUROPÄISCHE PATENTANMELDUNG NR.**

EP 22 18 6943

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-12-2022

| Im Recherchenbericht angeführtes Patentdokument | Datum der Veröffentlichung | Mitglied(er) der Patentfamilie | Datum der Veröffentlichung |
|---|---|---|---|
| WO 2011126371 A2 | 13-10-2011 | EP 2556030 A2 | 13-02-2013 |
| | | WO 2011126371 A2 | 13-10-2011 |
| CN 204563692 U | 19-08-2015 | KEINE | |
| US 2017291122 A1 | 12-10-2017 | US 2017291122 A1 | 12-10-2017 |
| | | US 2018236380 A1 | 23-08-2018 |
| | | US 2021268406 A1 | 02-09-2021 |
| US 2017128857 A1 | 11-05-2017 | KEINE | |
| WO 2011130321 A2 | 20-10-2011 | CA 2796117 A1 | 20-10-2011 |
| | | CN 102958575 A | 06-03-2013 |
| | | CN 106964176 A | 21-07-2017 |
| | | EP 2558179 A2 | 20-02-2013 |
| | | JP 5878164 B2 | 08-03-2016 |
| | | JP 6235051 B2 | 22-11-2017 |
| | | JP 2013523449 A | 17-06-2013 |
| | | JP 2016106026 A | 16-06-2016 |
| | | KR 20130103315 A | 23-09-2013 |
| | | US 2011278218 A1 | 17-11-2011 |
| | | US 2016052803 A1 | 25-02-2016 |
| | | WO 2011130321 A2 | 20-10-2011 |
| US 2011123392 A1 | 26-05-2011 | US 2011123392 A1 | 26-05-2011 |
| | | US 2014202876 A1 | 24-07-2014 |
| | | US 2016347628 A1 | 01-12-2016 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013138797 A